Europäisches Patentamt

European Patent Office

Office européen des brevets

(19)

(11) Veröffentlichungsnummer: **0 141 100**
**B1**

(12) **EUROPÄISCHE PATENTSCHRIFT**

(45) Veröffentlichungstag der Patentschrift: **02.05.90**

(51) Int. Cl.⁵: **A 61 K 49/02**

(21) Anmeldenummer: **84109914.6**

(22) Anmeldetag: **21.08.84**

(54) N-(4-Aminobenzoyl)-aminodicarbonsäuren zur Stabilisierung von Technetium-99m-Präparaten, stabilisierte Injektionspräparate und Verfahren zu ihrer Herstellung.

(30) Priorität: **30.08.83 DE 3331159**

(43) Veröffentlichungstag der Anmeldung:
**15.05.85 Patentblatt 85/20**

(45) Bekanntmachung des Hinweises auf die Patenterteilung:
**02.05.90 Patentblatt 90/18**

(84) Benannte Vertragsstaaten:
**AT BE CH DE FR GB IT LI**

(56) Entgegenhaltungen:
**EP-A-0 078 642**
**DE-A-2 617 922**

(73) Patentinhaber: **HOECHST AKTIENGESELLSCHAFT**
**Postfach 80 03 20**
**D-6230 Frankfurt am Main 80 (DE)**

(72) Erfinder: **Schwarz, Alexander, Dr.**
**Wiesenring 61**
**D-6093 Flörsheim am Main (DE)**

Courier Press, Leamington Spa, England.

# EP 0 141 100 B1

**Beschreibung**

Die vorliegende Erfindung betrifft Substanzen, durch die Technetium-99m-haltige Injektionslösungen stabilisiert werden können, die zur Knochenszintigraphie verwendet werden.

In der nuklearmedizinischen Diagnostik ist Technetium-99m wegen seiner günstigen physikalischen Eigenschaften (kurze Halbwertszeit von 6 Stunden, $\gamma$-Energie von 140 keV und Fehlen einer Korpuskularstrahlung) und der damit verbundenen geringen Strahlenbelastung zum wichtigsten Radionuklid geworden.

Technetium-99m, das sich aus Nuklidgeneratoren gewinnen läßt, liegt zunächst als Pertechnetat vor, das zur szintigraphischen Abbildung der Schilddrüse und des Gehirns geeignet ist. Die Szintigraphie anderer Organe mittels Technetium-99m gelingt mit Hilfe bestimmter "Transportsubstanzen", die einerseits in der Lage sind, Technetium zu binden, und andererseits das Radionuklid zim Zielorgan mit hoher Selektivität anzureichern. Zur Markierung dieser organspezifischen "Transportsubstanz" mit Technetium-99m muß das aus dem Nuklidgenerator eluierte Pertechnetat zuerst in eine niedrigere Oxidationsstufe übergeführt werden. In dieser reduzierten Form bildet Technetium mit der organspezifischen Substanz mehr oder weniger stabile Verbindung. Die Reduktion des Pertechnetats ($TcO_4^-$) erreicht man entweder durch chemische Reduktionsmittel oder durch elektrolytische Methoden. In der Praxis verwendet man fast ausschließlich Zinn-II-Salze, wie Zinn-II-chlorid, -fluorid, -oxid oder -tartrat, wobei besonders das Chlorid bevorzugt wird.

Das Zinn-II-Salz liegt im allgemeinen zusammen mit der organspezifischen Substanz in lyophilisierter Form vor, wodurch diese Präparate haltbar gemacht und monatelang unverändert aufbewahrt werden können. In der Klinik muß der Anwender zur Herstellung des Tc-99m-markierten Injektionspräparats dann nur eine aus dem Nuklidgenerator eluierte sterile Lösung des Pertechnetats mit der benötigten Tc-99m-Aktivität zusetzen.

Diese Injektionslösungen sind im Gegensatz zum noch nicht markierten, aus organspezifischer Substanz und Zinn-II-Salz bestehenden Lyophilisat, nur einige Stunden haltbar. Das hängt weniger mit der relativ kurzen Halbwertszeit des Technetium-99m als mit der Stabilität der Zinn-II-Ionen in der Lösung zusammen. Es ist bekannt, daß Zinn-II-Verbindungen in neutraler wäßriger Lösung relativ rasch hydrolysiert werden. Da die Injektionslösungen physiologisch verträglich sein sollen, liegt ihr pH-Wert im allgemeinen zwischen 5 und 8, d.h. in einem Bereich, in dem Zinn-II nicht besonders stabil ist.

In den Tc-99m-Präparaten muß aber die organspezifische Substanz gegenüber dem als reduzierendem Agens wirkenden Zinn-II immer im Überschuß vorliegen, da die diagnostische Brauchbarkeit des Radiopharmakons vom Mengenverhältnis der Komponenten entscheidend beeinflußt wird. Ist der Zinn-II-Gehalt in einer Markierungseinheit zu hoch, dann kann unter Umständen nicht mehr alles Zinn komplex gebunden sein, und es kommt zur Bildung von reduziertem, jedoch nicht am Wirkstoff gebundenen Technetium. Diese als "Kolloid" bezeichnete Verunreinigung bildet sich dadurch, daß Zinn-Ionen beim pH-Wert der Eluate, der in der Regel zwischen 5 und 6 liegt, schwer lösliches Zinnhydroxid bilden, das in kolloidaler Form vorliegt. Das bei der Reduktion unter diesen Bedingungen entstehende Technetiumdioxid fällt zusammen mit dem Zinhydroxid aus, und es kommt zu einer erhöhten Anreicherung von Technetium-99m in Leber und Milz.

Umgekehrt würde, wenn die Menge an Zinn-II in der Injektionslösung nicht ausreichend wäre, noch Pertechnetat vorhanden sein, das die szintigraphische Organdarstellung durch Anreicherung im Magen und in der Schilddrüse stören kann. Folgende Näherungsrechnung zeigt, daß üblicherweise in einer Tc-99m-Markierungseinheit ein genügend hoher Überschuß an Zinn-II vorhanden ist, durch den das zugesetzte Pertechnetat quantitativ reduziert wird. 37 MBq (1 mCi) Tc-99m enthalten ca. $10^{12}$ Atome, entsprechend einer Menge von $1,92 \times 10^{-6}$ µMol oder 190 pg. Geht man davon aus, daß je nach Präparat bei der Markierung 185—7.400 MBq (5—200 mCi) eingesetzt werden, dann finden die chemischen Reaktionen mit Technetiummengen von einigen ng statt.

Der in der europäischen Patentschrift 00 02 485 beschriebenen Markierungseinheit für die Skelettszintigraphie, die aus 13 mg 3,3 - Diphosphono - 1,2 - propandicarbonsäure - tetra - natriumsalz als organspezifischer Substanz und 0,2 mg Zinn-II als reduzierendem Agens besteht, werden 10 ml Generatoreluat mit einer Aktivitätskonzentration von 740 MBq (20 mCi) pro ml zugesetzt. Die injektionsfertige Lösung enthält so mit den 7.400 MBq (200 mCi) $4 \times 10^{-4}$ µMol bzw. 40 ng Tc-99m. Das aus einem Nuklidgenerator gewonnene Technetium-99m ist jedoch nicht trägerfrei, da einerseits Molybdän-99 zu 14% direkt zu Tc-99 zerfällt und andererseits aus dem gebildeten Tc-99m ständig durch radioaktiven Zerfall Tc-99 entsteht. Bei täglicher Elution liegen pro Atom Tc-99m noch rund 2,5 Atome Tc-99 vor, die an allen chemischen Prozesen genauso teilnehmen wie Tc-99m. Das heißt, ein Teil des Zinn-II in der Markierungseinheit wird zur Reduktion des diagnostisch nicht nutzbaren Tc-99m-Pertechnetats verbraucht.

Im obigen Beispiel liegen demnach die drei Komponenten des injektionsfertigen Radiopharmakons Technetium (1,4 nMol), Zinn (1,7 µMol) und 3,3 - Diphosphono - 1,2 - propandicarbonsäure - tetranatriumsalz (34 µMol) im molaren Verhältnis 1:1.200:24.00 vor. Da für die Reduktion des Pertechnetats zum positiv 4-werdenden Technetium 3 Atome Zinn-II pro 2 Atomen Tc-VII benötigt werden, stellen die in der Markierungseinheit vorhandenen 0,2 mg Zinn-II einen 800-fachen Überschuß dar.

Dieser hohe Überschuß des Zinn-II gegenüber Technetium, der bei fast allen Tc-99m-Präparaten die Regel ist, garantiert eine $TcO_4^-$-freie Injektionslösung, die meistens auch mehrere Stunden stabil ist.

2

Voraussetzung für die Stabilität des Tc-99m-Injektionspräparates ist die Anwesenheit von Zinn-II in der Lösung. Wird das Zinn-II in der Injektionslösung aber nach mehr oder weniger kurzer Standzeit durch Hydrolyse verbraucht, dann kann unter dem Einfluß von geringen Mengen Luftsauerstoff aus dem reduzierten Technetium in der Lösung durch Reoxidation wieder Pertechnetat entstehen.

Man hat versucht, durch Zusatz von Antioxidantien eine Reoxidation des Technetium in Injektionspräparaten zu verhindern. So wurden zum Beispiel Ascorbinsäure, Gentisinsäure bzw. Nitrit zur Stabilisierung von Tc-99m-Präparaten vorgeschlagen (vgl. z.B. deutsche Offenlegungsschrift 26 18 337, europäische Patentanmeldung mit der Veröffentlichungs-Nr. 00 04 684 und 00 46 067). Davon werden Gentisin- und Ascorbinsäure bereits in manchen Handelspräparaten als Stabilisatorzusätze verwendet.

Untersuchungen mit diesen Substanzen zeigten jedoch, daß ihre Wirksamkeit bei Verwendung hoher Tc-99m-Aktivitätskonzentrationen gering ist. Manche Tc-99m-Präparate, vor allem die zur Skelettszintigraphie verwendeten Diphosphonate, werden oft unter Verwendung hoher Aktivitätskonzentrationen markiert. Durch Verteilung des Inhalts einer Markierungseinheit auf möglichst viele Patienten versucht man, die Kosten pro Untersuchung zu senken. Dafür müssen hohe Tc-99m-Aktivitäten pro Markierungseinheit zugesetzt werden, und es wird eine längere Anwendungsdauer der Injektionslösung, möglichst über einen vollen Arbeitstag, angestrebt.

Wie umfangreiche eigene Untersuchungen ergaben, nimmt die Stabilität einer Tc-99m-Injektionslösung bei Zugabe steigender Tc-99m-Aktivitäten ab. So wurde bei dem oben erwähten Knochendiagnostikum 3,3 - Diphosphono - 1,2 - propandicarbonsäure (DPD) gefunden (Tab. 1), daß bei Zugabe von 3.700 MBq.

TABELLE 1

Einfluß der zugesetzten Tc-99m-Aktivitätsmenge auf den Zinn-II-Gehalt und den Pertechnetatanteil in der Markierungseinheit 3,3-Diphosphono-1,2-propandicarbonsäure (DPD) in Abhängigkeit von der Zeit nach Präparation mit je 5 ml Eluat

| MBq | Zinn-II-Gehalt *) nach Präparation (h) in µg | | | | Pertechnetatanteil **) in der Lösung nach Präparation (h) in % | | | |
|---|---|---|---|---|---|---|---|---|
| | 0 | 2 | 5 | 8 | 0 | 2 | 5 | 8 |
| 1.850 | 200 | 168 | 137 | 108 | <1 | <1 | <1 | <1 |
| 3.700 | 200 | 152 | 119 | 88 | <1 | <1 | <1 | <1 |
| 7.400 | 200 | 136 | 84 | 30 | <1 | <1 | <1 | 2 |
| 11.100 | 200 | 115 | 48 | <10 | <1 | <1 | <1 | 4 |
| 14.800 | 200 | 96 | <10 | — | <1 | <1 | 3 | 8 |

*) Zinn-II-Bestimmung durch jodometrische Titration.
**) Messung des Tc-99m-Pertechnetatanteils durch Papierchromatographie (Whatman-Papier No. 1; Methanol:Wasser=8:2).

(100 mCi) pro Markierungseinheit nach 8-stündiger Standzeit noch genügend Zinn-II-Ionen vorhanden waren, um das Auftreten von Pertechnetat in der Lösung zu verhindern. Dagegen zeigte sich, daß bei Verwendung von 14.800 MBq (400 mCi) in 5 ml Generatoreluat bereits 5 Stunden nach der Präparation alles Zinn-II verbraucht und in der Lösung naturgemäß freies Pertechnetat nachweisbar war.

Die DE—A—26 17 922 beschreibt ein Verfahren zur Herstellung von lyophilisierten Produkten, die zum Markieren mit Radionukliden geeignet sind. Zur Vermeidung der Verschlechterung der physikalischen und chemischen Eigenschaften der Teilchen während der Lyophilisierung und danach wird, der Dispersion zusätzlich ein Schutzmittel zugesetzt. Als geeignete Schutzmittel werden Aminosäuren und in diesem Zusammenhang auch Asparaginsäure und Glutaminsäure genannt.

Unter dem Einfluß hoher Tc-99m-Aktivitäten kommt es in der Injektionslösung zu einer beschleunigten strahlenchemischen Oxidation der reduzierenden Zinn-II-Ionen. Bei der Suche nach wirsameren Stabilisatoren wurden in den substituierten Aminodicarbonsäuren N - (4 - Aminobenzoyl) - L - asparaginsäure und N - (4 - Aminobenzoyl) - L - glutaminsäure überraschend Substanzen gefunden, durch die auch hohe Tc-99m-Aktivitätskonzentratonen enthaltende Injektionslösungen über einen längeren Zeitraum nach Präparierung haltbar gemacht werden können.

Die Erfinding betrifft daher die N - (4 - Aminobenzoyl) - asparaginsäure und -glutaminsäure, ihre physiologisch verträglichen Salze sowie ihre Ester als Stabilisatoren für Zinn-II-Verbindungen enthaltende Tc-99m-Injektionslösungen zur Knochenszintigraphie sowie Verfahren zur Herstellung stabilisierter, T-99m-markierter Radiodiagnostika, bei denen N - (4 - Aminobenzoyl) - asparaginsäure bzw. -glutaminsäure oder ihre Ester als zusätzliche Komponenten entweder der inaktiven Markierungseinheit oder zusammen mit der Technetium-99m-Pertechnetatlösung der zu markierenden Substanz zugesetzt werden.

Bei Zusatz des Stabilisators zur inaktiven Markierungseinheit wird entweder der Stabilisator dem Gemisch aus organspezifischer Komponente und Zinn-II-Verbindung vor der Abfüllung zugesetzt und die erhaltene Lösungs portionsweise abgefüllt und lyophilisiert, oder der lyophilisierte Inhalt einer Abfüllung (Markierungseinheit) wird zuerst in stabilisatorhaltiger physiologischer Kochsalzlösung gelöst und dann durch Zugabe der Tc-99m-Pertechnetatlösung markiert. Da durch einen zu hohen Anteil der Stabilisatorkomponente die Organverteilung des Technetium-99m-Präparats verändert werden kann, ist es zweckmäßig, ein molares Verhältnis von Zinn-II zu Stabilisator von 1:10 nicht zu überschreiten. Verteilungsstudien an Ratten zeigten, daß bei einem molaren Verhältnis von Zinn-II zu N - (4 - Aminobenzoyl) - L - glutaminsäure von 1:10 bis 1:30, die als reduzierende bzw. stabilisierende Komponenten der knochenspezifischen Tc - 99m - Diphosphono - 1,2 - propandicarbonsäure (DPD) zugesetzt wurden, die Skelettspeicherung dieses Tc-99m-Komplexes deutlich auf Kosten einer verstärkten renalen Ausscheidung verringert war.

Optimal, und für die Stabilisierung auch bei Zugabe sehr hoher Tc-99m-Aktivitäten (bis zu 18.500 MBq) ausreichend, ist ein Molverhältnis Zinn-II zu N-substituierter Aminodicarbonsäure von 1:2 bis 1:6, so daß eine für die Anwendung beim Menschen geeignete Abfüllung (Markierungseinheit) sinnvollerweise zusätzlich 0,85—2,7 mg Stabilisator zu der organspezifischen Substanz enthält, wenn das ebenfalls darin enthaltende reduzierende Agens ($Sn^{2+}$) in einer Menge von rund 0,2 mg vorliegt.

Für die Herstellung einer Markierungseinheit werden als Stabilisatoren gemäß der Erfindung entweder die N - (4 - Aminobenzoyl) - asparaginsäure oder die N - (4 - Aminobenzoyl) - glutaminsäure, vorteilhafter ihre Natriumsalze eingesetzt, da sie in Wasser leichter löslich sind. Daneben eigenen sich auch die Dimethyl- bzw. Diethylester der N - (4 - Aminobenzoyl) - asparaginsäure bzw. -glutaminsäure.

Beispiel 1

Zu 4 ml Pertechnetatlösung, die 14.800 MBq (400 mCi) Tc-99m enthalten, gibt man 1 ml einer physiologischen NaCl-Lösung (pH ~7), in der 2 mg N - (4 - Aminobenzoyl) - L - glutaminsäurenatriumsalz gelöst sind. Diese Lösung wird einer lyophilisierten Markierungseinheit, bestehend aus 13 mg 3,3 - Diphosphono - 1,2 - propandicarbonsäure - tetranatriumsalz und 0,2 mg Zinn-II, zugesetzt. Nach 16-stündiger Standzeit wird mit physiologischer NaCl-Lösung auf eine Konzentration von 0,26 mg Diphosphonat/ml verdünnt und jeweils 0,05 ml (13 µg) pro Ratte i.v. appliziert. Parallel dazu wird das gleiche Präparat, jedoch ohne Zustatz der N - (4 - Aminobenzoyl) - L - glutaminsäure als Stabilisator, mit 14.800 MBq Tc-99m markiert, nach gleicher Standzeit verdünnt und in analoger Konzentration Ratten injiziert.

Die Organverteilung in jeweils 3 Ratten wurde 2 Stunden nach Injektion bestimmt. Folgende Werte (in % der applizierten Dosis, vgl. Tab. 2) zeigen den deutlichen Unterschied in der Organverteilung zwischen dem stabilisatorhaltigen und -freien Tc-99m-Präparat, wobei die erhöhten Anreicherungen in Magen und Schilddrüse auf die Anwesenheit von Pertechnetat in der Injektionslösung hinweisen.

TABELLE 2

| | Tc-99m-DPD | |
|---|---|---|
| | mit Stabilisator | ohne Stabilisator |
| Knochen/g | 4,22 | 3,92 |
| Blut/ml | 0,015 | 0,068 |
| Muskel/g | 0,005 | 0,040 |
| Leber | 0,22 | 0,58 |
| Lunge | 0,058 | 0,096 |
| Nieren | 0,44 | 1,20 |
| Darm | 1,11 | 3,26 |
| Magen | 0,29 | 2,86 |
| Schilddrüse | 0,017 | 0,061 |

Beispiel 2

Eine Markierungseinheit bestehend aus 7,2 mg Natriumpyrophosphat und 1,08 mg $SnCl_2$, wird in 2 ml physiologischer NaCl-Lösung (pH ~7), die N - (4 - Aminobenzoyl) - L - asparaginsäure - natriumsalz in einer Konzentration von 1 mg/ml enthält, gelöst. Unmittelbar darauf werden 8 ml Pertechnetatlösung mit 12.950 MBq (350 mCi) zugesetzt und anschließend in bestimmten Zeitintervallen Proben aus dem Markierungsansatz entnommen, die papierchromatographisch auf freies Pertechnetat geprüft werden. Die Prüfung erfolgt auf Whatman-1-Papier unter Verwendung von Methanol:Wasser=8:2 (v/v) als mobiler Phase. Das mit einem Rf-Wert von 0,6 vom Tc-99m-Pyrophosphat (Rf=0) abgetrennte Pertechnetat wird mit Hilfe eines Radiochromatographen bestimmt. Ein unter gleichen Bedingungen markiertes Pyrophosphat, dem jedoch kein Stabilisator zugesetzt wurde, enthält bereits, wie aus der Tabelle 3 ersichtlich, nach relativ kurzer Standzeit Pertechnetat:

TABELLE 3

| Zeit nach Präparation (h) | % Pertechnetat in Tc-99m-Pyrophosphat | |
|---|---|---|
| | mit Stabilisator | ohne Stabilisator |
| 0,5 | <1 | <1 |
| 2 | <1 | <1 |
| 4 | <1 | 1 |
| 6 | <1 | 3 |
| 8 | <1 | 7 |
| 12 | <1 | 10 |

Beispiel 3

2 g Methylendiphosphonsäure (MDP) und 268 mg N - (4 - Aminobenzoyl) - L - glutaminsäure - diethylester werden zusammen in 3 ml bidest. Wasser gelöst und mit ca. 27 ml 1 N-Natronlauge auf einen pH-Wert von 6,5 eingestellt. Unter Luftausschluß werden 56 mg Zinn-II-oxid der Lösung zugesetzt. Nach vollständiger Auflösung des SnO wird mit 70 ml Wasser verdünnt und die sterilfiltrierte Lösung in 0,5 ml-Portionen in mit flüssigen Stickstoff vorgefrorene Rollrandfläschchen vereinzelt und gefriergetrocknet. Die Abfüllungen werden mit Stickstoff überlagert und verschlossen.

Durch jodometrische Titration untersuchte Proben zeigten nach mehrwöchiger Lagerzeit einen unveränderten Gehalt von 0,2 mg Zinn-II pro Abfüllung. Das mit Tc-99m-Pertechnetatlösungen hoher Aktivitätskonzentrationen versetzte Präparat war auch nach 8-stündiger Standzeit frei von Pertechnetat und zur szintigraphischen Skelettdarstellung geeignet.

Beispiel 4

Unter luftfreien Bedingungen löst man bei Raumtemperatur in 26 ml 2 N-Natronlauge 90 mg reinstes Zinn-II-oxid. In die Natriumstannitlösung läßt man unter Rühen eine Lösung, bestehend aus 4 g 3,3 - Diphosphono - 1,2 - propandicarbonsäure (DPD) und 356 mg N - (4 - Aminobenzoyl) - L - aminoglutaminsäure in 20 ml bidest. Wasser, zufließen. Die klare Lösung wird mit sterilem Wasser auf ein Gesamtvolumen von 200 ml verdünnt und anschließend in 0,5 ml Portionen in mit flüssigen Stickstoff vorgekühlte Rollrandfläschchen abgefüllt. Nach der Gefriertrocknung werden die Gläschen unter Vakuum verschlossen.

Es wurden, wie unter Beispiel 1 beschrieben, Tierversuche mit dem Tc-99m-DPD durchgeführt, wobei nach 16-stündiger Standzeit der Injektionslösungen und 2 Stunden nach i.v.-Applikation des Präparats folgende Ergebnisse erhalten wurden (in % der applizierten Dosis):

| Knochen | Leber | Nieren | Schilddrüse | Magen | Blut |
|---|---|---|---|---|---|
| 45,3 | 0,26 | 0,77 | 0,018 | 0,22 | 0,36 |

**Patentansprüche**

1. N - (4 - Aminobenzoyl) - asparaginsäure oder -glutaminsäure, ihre physiologisch verträglichen Salze oder ihre Ester zur Verwendung als Stabilisatoren für Tc-99m-Injektionslösungen zur Knochenszintigraphie, die eine Zinn-II-Verbindung als reduzierende Komponente und eine organspezifische Substanz enthalten.

5

2. Tc-99m-Injektionspräparat enthaltend eine Zinn-II-Verbindung als reduzierende Komponente und eine organspezifische Substanz, dadurch gekennzeichnet, daß es als Stabilisator N - (4 - Aminobenzoyl) - asparaginsäure oder N - (4 - Aminobenzoyl) - glutaminsäure oder ein physiologisch verträgliches Salz oder einen Dimethyl-bzw. Diethylester dieser Verbindungen enthält.

3. Verfahren zur Herstellung eines Injektionspräparates gemäß Anspruch 2, dadurch gekennzeichnet, daß der Stabilisator dem Gemisch aus organspezifischer Substanz und Zinn-II-Verbindung vor der Abfüllung zugesetzt und die erhaltene Lösung portionsweise abgefüllt sowie lyophilisiert wird.

4. Verfahren zur Herstellung eines Injektionspräparates gemäß Anspruch 2, dadurch gekennzeichnet, daß man den Stabilisator der Tc-99m-Pertechnetatlösung zusetzt und mit Hilfe dieser stabilisatorhaltigen Lösung das Diagnostikum radioaktiv markiert.

5. Verfahren zur Herstellung eines Injektionspräparates gemäß Anspruch 2, dadurch gekennzeichnet, daß der lyophilisierte Inhalt einer Abfüllung zuerst in stabilisatorhaltigen physiologischer Kochsalzlösung gelöst und dann durch Zugabe der Tc-99m-Pertechnetatlösung markiert wird.

6. Verfahren nach Anspruch 3, dadurch gekennzeichnet, daß N - (4 - Aminobenzoyl) - asparaginsäure oder -glutaminsäure oder eines ihrer Salze der einer ihrer Ester mit dem als Reduktionsmittel vorhandenen Zinn-II in einem Molverhältnis von 10:1 bis 2:1, vorzugsweise 6:1 bis 2:1, zugesetzt wird.

7. Verfahren nach Anspruch 3, dadurch gekennzeichnet, daß einer Abfüllung 0,1 bis 10 mg, vorzugsweise 0,5 bis 3 mg, N - (4 - Aminobenzoyl) - asparaginsäure oder N - (4 - Aminobenzoyl) - glutaminsäure oder das entsprechende Natriumsalz zugesetzt wird.

## Revendications

1. Utilisation d'acide N - (4 - aminobenzoyl) - aspartique ou d'acide N - (4 - aminobenzoyl) - glutamique, de leurs sels physiologiquement acceptables ou de leurs esters comme stabilisants de solutions injectables de Tc-99m pour la scintigraphie osseuse, solutions renfermant un composé d'étain-II comme composant réducteur et une substance organospécifique.

2. Préparation injectable de Tc-99m renfermant un composé d'étain-II comme composant réducteur et une substance organospécifique, caractérisée en ce qu'elle contient, comme stabilisant, de l'acide N - (4 - aminobenzoyl) - aspartique ou de l'acide N - (4 - aminobenzoyl) - glutamique, ou un sel physiologiquement acceptable ou un ester diméthylique ou diéthylique de ces composés.

3. Procédé de fabrication d'une préparation injectable selon la revendication 2, caractérisé en ce que le stabilisant est ajouté au mélange de substance organospécifique et de composé d'étain-II avant conditionnement, et que la solution obtenue est conditionnée et lyophilisée par portions.

4. Procédé de fabrication d'une préparation injectable selon la revendication 2, caractérisé en ce que l'on ajoute le stabilisant à la solution de Pertechnétate-Tc-99m et que l'on effectue le marquage radioactif du produit de diagnostic à l'aide de cette solution renfermant le stabilisant.

5. Procédé de fabrication d'une préparation injectable selon la revendication 2, caractérisé en ce que l'on dissout d'abord le contenu lyophilisé d'un conditionnement dans une solution physiologique de chlorure de sodium renfermant le stabilisant, et que l'on effectue ensuite le marquage par addition de la solution de Pertechnétate-Tc-99m.

6. Procédé selon la revendication 3, caractérisé en ce que l'on ajoute l'acide N - (4 - aminobenzoyl) - aspartique ou l'acide N - (4 - aminobenzoyl) - glutamique, ou un de leurs sels ou un de leurs esters, avec le composé d'étain-II comme composant réducteur, en un rapport molaire compris entre 10:1 et 2:1, de préférence entre 6:1 et 2:1.

7. Procédé selon la revendication 3, caractérisé en ce que l'on ajoute à un conditionnement de 0,1 à 10 mg, de préférence 0,5 à 3 mg d'acide N - (4 - aminobenzoyl) - aspartique ou d'acide N - (4 - aminobenzoyl) - glutamique, ou du sel de sodium correspondant.

## Claims

1. N - (4 - Aminobenzoyl) - aspartic or -glutamic acid, their physiologically acceptable salts or their esters for use as stabilizers for Tc-99m-injection-solutions for bone scintigraphy, which solutions contain a tin-II compound as a reducing component and an organ-specific substance.

2. A Tc-99m-injection preparation, containing a tin-II compound as a reducing component and an organ-specific substance, which comprises, as a stabilizer, N - (4 - aminobenzoyl) - aspartic acid or N - (4 - aminobenzoyl) - glutamic acid or a physiologically acceptable salt or a dimethyl or diethyl ester of said compounds.

3. A process for producing an injection preparation as claimed in claim 2, which comprises adding the stabilizer to the mixture of organ-specific substance and tin-II compound before filling and filling portions of the solution obtained into containers, and lyophilizing them.

4. A process for producing an injection preparation as claimed in claim 2, which comprises adding the stabilizer to the Tc-99m-pertechnetate solution and radioactively labelling the diagnostic agent by means of this stabilizer-containing solution.

5. A process for producing an injection preparation as claimed in claim 2, which comprises first

6

dissolving the lyophilized content of a filled container in stabilizer-containing physiological saline and then labelling it by adding the Tc-99m-pertechnetate solution.

6. The process as claimed in claim 3, wherein N - (4 - aminobenzoyl) - aspartic acid or -glutamic acid or one of their salts or one of their esters is added together with tin-II, present as a reducing agent, in a molar ratio of 10:1 to 2:1, preferably 6:1 to 2:1.

7. The process as claimed in claim 3, wherein 0.1 to 10 mg, preferably 0.5 to 3 mg, of N - (4 - aminobenzoyl) - aspartic acid or N - (4 - aminobenzoyl) - glutamic acid or the corresponding sodium salt are added to one filled container.